# EUROPÄISCHE PATENTSCHRIFT

(11) **EP 2 454 242 B1**
(45) Veröffentlichungstag und Bekanntmachung des Hinweises auf die Patenterteilung: **15.10.2014**
(21) Anmeldenummer: 10732316.4
(22) Anmeldetag: 14.07.2010
(51) Int. Cl.: C07D 249/08

(54) **VERFAHREN ZUR HERSTELLUNG VON CHIRALEN 3-TRIAZOLYL-SULFOXID-DERIVATEN**
METHOD FOR PRODUCING CHIRAL 3-TRIAZOLYL-SULFOXIDE DERIVATIVES
PROCÉDÉ DE PRÉPARATION DE DÉRIVÉS 3-TRIAZOLYL-SULFOXYDE CHIRAUX

(30) Priorität: 16.07.2009 DE 102009027771
(43) Veröffentlichungstag der Anmeldung: 23.05.2012
(73) Patentinhaber: Bayer CropScience AG, 40789 Monheim (DE)
(72) Erfinder: ANTONS, Stefan, 51373 Leverkusen (DE); LUI, Norbert, 51519 Odenthal (DE); MORADI, Wahed, Ahmed, 40789 Monheim (DE)
(86) Internationale Anmeldenummer: PCT/EP2010/004288
(87) Internationale Veröffentlichungsnummer: WO 2011/006646

(56) Entgegenhaltungen:
- EP-A1- 1 076 053
- WO-A1-2006/040635
- CN-A- 101 012 141
- CN-A- 101 429 192
- FR-A1- 2 863 611
- FR-A1- 2 876 101
- BRYLIAKOV, K.P.: "Transition Metal Catalyzed Asymmetric Oxidation of Sulfides" CURRENT ORGANIC CHEMISTRY, Bd. 12, 2008, Seiten 386-404, XP009138659

## Beschreibung

Die vorliegende Erfindung betrifft ein katalytisches Verfahren zur Herstellung von 3-Triazolyl-Sulfoxid-Derivaten in enantiomerenreiner oder in einer enantiomeren-angereicherten Form.

Die chemische Synthese von 3-Triazolyl-Sulfoxiden ist in der Literatur beschrieben, führt aber zu einer racemischen Mischung (WO 1999/055668).

Enantiomerenreine chirale Sulfoxide und entsprechende Derivate sind in der pharmazeutischen und agrochemischen Industrie von großer Bedeutung. Derartige Verbindungen können weiterverarbeitet werden, um ausschließlich das biologisch wirksame Enantiomer eines Arzneimittels oder chemischen Pflanzenschutzmittels bereitzustellen. Dieses schließt nicht nur Abfall beim Herstellungsverfahren aus, sondern umgeht auch potentiell schädliche Nebenwirkungen, die aus dem unerwünschten Enantiomer entstehen können (Nugent et al., Science 1993, 259, 479; Noyori et al., CHEMTECH 1992, 22, 360).

Die enantioselektive Synthese von chiralen Sulfoxiden ist in der Literatur beschrieben. Übersichtsartikel, die diese Methodik beschreiben, findet man beispielsweise in H. B. Kagan in " Catalytic Asymmetric Synthesis"; I. Ed. VCH: New York 1993, 203-226; Ojima N. Khiar in Chem. Rev. 2003, 103, 3651-3705; K. P. Bryliakov in Current Organic Chemistry 2008, 12, 386-404. Neben den klassisch metallkatalysierten Methoden zur Synthese von chiralen Sulfoxiden werden in der Literatur auch enzymatische und chromatographische Verfahren beschrieben (K. Kaber in "Biotransformations in Organic Synthesis", Springer Ed. 3rd ed. 1997**;** H. L. Holland, Nat. Prod. Rep., 2001, 18, 171-181). Dabei sind die enzymatischen Methoden überwiegend substratspezifisch und darüberhinaus ist die technische Realisierung sehr teuer und aufwendig. Beispielsweise sind Monooxygenasen und Peroxidasen wichtige Enzymklassen, die in der Lage sind, eine Vielzahl von Sulfiden in Sulfoxide zu katalysieren (S. Colonna et al., Tetrahedron Asymmetry 1993, 4, 1981). Jedoch wurde gezeigt, daß das stereochemische Resultat der enzymatischen Oxidation stark von der Sulfidstruktur abhängig ist.

Ein häufig angewandtes Verfahren zur enantioselektiven Oxidation von Thioethern ist die von Kagan modifizierte Methode der bekannten Sharpless-Epoxidierung mit chiralen Titankomplexen (J. Am. Chem. Soc. 1984, 106, 8188-8193). Dabei wird der chirale Titankomplex, bestehend aus Ti(OPrⁱ)₄ und (+)- oder (-)-Diethyltartrat (DET) mit einem Äquivalent Wasser "desaktiviert" und katalysiert die enantioselektive Sulfidoxidation von Arylalkylsulfiden. Allerdings wurden gute Ergebnisse mit dem Kagan-Reagenz mit einem Mischungsverhältnis Ti(OPrⁱ)₄/DET/H₂O = 1:2:1 und einem organischen Peroxid (z. B. *tert*.-Butylhydroperoxid) erzielt. Die gute Enantioselektivität der beschriebenen Titankomplexe wird von einer niedrigen katalytischen Aktivität begleitet, die das nötige Mengenverhältnis zwischen Substrat und Katalysator beschreibt. Mittels dieses Verfahrens kann die direkte Oxidation von einfachen Arylalkylsulfiden, wie z. B. Arylmethylsulfiden zu optisch aktiven Sulfoxiden erreicht werden. Es wurde gefunden, daß die asymmetrische Oxidation von beispielsweise funktionalisierten Alkylsulfiden unter diesen Bedingungen mit mäßiger Enantioselektivität erfolgt.

Pasini et al. konnten zwar mit geringen Mengen chiralen Oxotitan(IV)-Schiff'sche Basen und Wasserstoffperoxid Phenylmethylsulfid oxidieren, jedoch mit schlechten Enantiomerenüberschüssen (ee-Werte<20%) (Gaz. Chim. Ital. 1986, 116, 35-40). Ähnliche Erfahrungen beschreiben Colona et al. mit chiralen Titankomplexen von *N*-Salicyl-*L*-aminosäuren (Org. Bioorg. Chem. 1987, 71-71). Des Weiteren resultieren aus titankatalysierten Verfahren sehr aufwendige Aufarbeitungen, was für einen ökonomischen Prozess im technischen Maßstab sehr nachteilig ist.

Eine weitere Methode basiert auf Vanadium(IV)-Schiff sche Basen als effiziente Katalysatoren für die Sulfidoxidation. Der chirale Katalysator wird *in*-*situ* aus VO(acac)₂ mit einer Schiff'schen Base von chiralen Aminoalkoholen hergestellt (Synlett 1998, 12, 1327-1328; Euro. J. Chem. 2009, 2607-2610). Allerdings ist diese Methode auf einfache und nicht fluorierte Arylalkylthioether wie beispielsweise p-Tolylmethylsulfid beschränkt.

Bisher wurden die Enantiomeren von 3-Triazolyl-Sulfoxiden, die nach literaturbekannten Verfahren racemisch anfielen, durch eine aufwendige Trennung mittels HPLC an chiralen Phasen erhalten. Die chromatographische Trennung von Enantiomeren an chiralen stationären Phasen ist jedoch in der Regel für größere Wirkstoffmengen nicht geeignet, sondern dient lediglich zur Bereitstellung kleinerer Mengen. Des Weiteren ist die Nutzung der HPLC an chiralen Phasen aufgrund des hohen Preises dieser Materialien und des erheblichen zeitlichen Aufwandes, insbesondere im präparativen Maßstab extrem kosten-intensiv.

Es bestand daher ein dringender Bedarf an einem katalytischen Verfahren, welches insbesondere im technischen Maßstab durchführbar ist. Der Erfindung liegt somit die Aufgabe zugrunde, ein solches katalytisches Verfahren bereitzustellen, das neben der technischen Machbarkeit, Kostengünstigkeit, gute Ausbeuten und eine Variation des Enantiomerenverhältnisses gewährleistet.

Im Hinblick auf die vorstehend geschilderten Nachteile und Probleme besteht dringend Bedarf für ein vereinfachtes, technisch und ökonomisch durchführbares, katalytisches Verfahren zur enantioselektiven Sulfidoxidation von substituierten, fluorierten 3-Triazolyl-Sulfoxid-Derivaten. Die mit diesem angestrebten Verfahren erhältlichen 3-Triazolyl-Sulfoxid-Derivate sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden wie chiraler Chromatographie ermöglichen.

Die Trennung der Enantiomeren und auch die Synthese von 3-Triazolyl-Sulfoxid Derivaten, die eine chirale Sulfoxid-Gruppe in enantiomerenreiner Form oder in einer enantiomerenangereicherten Form besitzen, ist bisher nicht beschrieben.

Die Aufgabe wurde gemäß der vorliegenden Erfindung durch ein Verfahren zum Herstellen von 3-Triazolyl-Sulfoxid-Derivaten der allgemeinen Formel (I) gelöst, 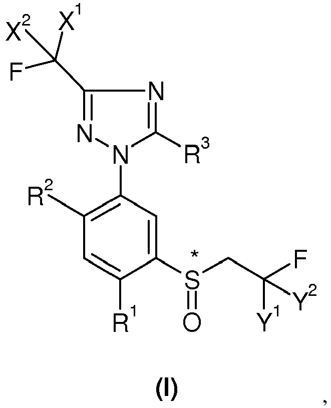 in welcher
X¹ und X² unabhängig voneinander für Fluor, Chlor, Brom, Wasserstoff, (C₁-C₁₂)Alkyl, (C₁-C₁₂)Halogenalkyl stehen,
Y¹ und Y² unabhängig voneinander für Fluor, Chlor, Brom, Wasserstoff, (C₁-C₁₂)Alkyl, (C₁-C₁₂)Halogenalkyl stehen,
R¹ und R² unabhängig voneinander für Wasserstoff, (C₁-C₁₂)Alkyl, (C₁-C₁₂)Halogenalkyl, Cyano, Halogen, Nitro stehen,
R³ für Wasserstoff, (C₁-C₁₂)Alkyl, Amino, Nitro, NH(CO)(C₁-C₁₂)Alkyl, N=CR'R steht,
wobei R, R' unabhängig voneinander für Wasserstoff, (C₁-C₁₂)Alkyl, Aryl stehen,
dadurch gekennzeichnet, daß man ein Sulfid der Formel (II) 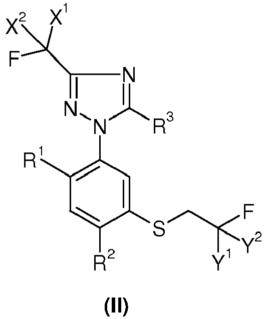 in welcher X¹, X², Y¹, Y² , R¹, R² und R³ die oben genannten Bedeutungen haben,
in Gegenwart eines chiralen Katalysators und eines Oxidationsmittels umsetzt,
wobei das Oxidationsmittel ausgewählt ist aus Wasserstoffperoxid, Alkylhydroperoxid und Arylalkylhydroperoxid und
wobei der chirale Katalysator ein chiraler Metall-Ligand-Komplex ist und das Metall ein Übergangsmetall ist.

Bevorzugte, besonders bevorzugte und ganz besonders bevorzugte Bedeutungen der in den oben erwähnten allgemeinen Formel (I) aufgeführten Reste X¹, X² , Y¹, Y², R¹, R² und R³ werden im Folgenden erläutert.
X¹ , X² , Y¹ und Y² stehen unabhängig voneinander bevorzugt für Fluor, Chlor, Wasserstoff, (C₁-C₁₂)Alkyl, (C₁-C₁₂)Halogenalkyl,
R¹ und R² stehen unabhängig voneinander bevorzugt für Fluor, Chlor, Wasserstoff, (C₁-C₁₂)Alkyl,
R³ steht bevorzugt für Wasserstoff, (C₁-C₁₂)Alkyl, Amino,
X¹ und X², Y¹ und Y² stehen unabhängig voneinander besonders bevorzugt für Fluor, Chlor, Wasserstoff, (C₁-C₁₂)Halogenalkyl,
R¹ und R² stehen unabhängig voneinander besonders bevorzugt für Fluor, Wasserstoff, (C₁-C₆)Alkyl,
R³ steht besonders bevorzugt für Wasserstoff, Amino,
X¹ und X², Y¹ und Y² stehen unabhängig voneinander ganz besonders bevorzugt für Fluor, Wasserstoff, (C₁-C₆)Halogenalkyl.
R¹ und R² stehen unabhängig voneinander ganz besonders bevorzugt für Fluor, Methyl,
R³ steht ganz besonders bevorzugt für Wasserstoff.

Überraschenderweise lassen sich die chiralen 3-Triazolyl-Sulfoxid-Derivate der Formel (I) unter den erfindungsgemäßen Bedingungen mit guten Ausbeuten in hoher Reinheit herstellen, womit das erfindungsgemäße Verfahren die im Zusammenhang mit dem Stand der Technik beschriebenen Nachteile nicht aufweist.

Verbindungen der Formel (I) entstehen nach dem erfindungsgemäßen Verfahren, je nach Herstellungsbedingungen, in einem Enantiomerenverhältnis von 50,5:49,5 bis 99,5:0,5 (+):(-)-Enantiomer oder(-):(+)-Enantiomer.

Die Enantiomerenreinheit kann, falls notwendig, nach unterschiedlichen Verfahren erhöht werden. Solche Verfahren sind dem Fachmann bekannt und schließen insbesondere die bevorzugte Kristallisation aus einem organischen Lösungsmittel oder einem Gemisch aus organischem Lösungsmittel mit Wasser ein.

Das erfindungsgemäße Verfahren kann anhand des folgenden Schemas (I) erläutert werden: 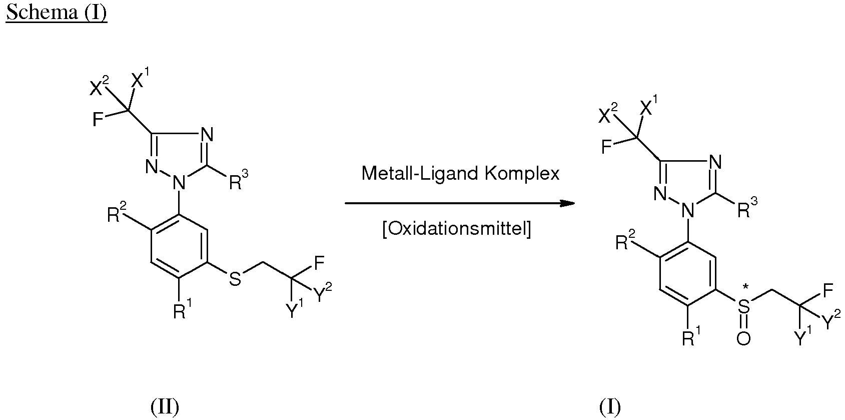 wobei X¹, X², Y¹, Y², R¹, R², R³ die oben angegebenen Bedeutungen haben.

### Allgemeine Definitionen

Im Zusammenhang mit der vorliegenden Erfindung umfasst der Begriff Halogene (Hal), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen.

Die Definition C₁-C₁₂-Alkyl umfasst den größten hierin definierten Bereich für einen AlkylGruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S, aufweisen können.

Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Alkylaryl-Gruppen sind im Zusammenhang mit der vorliegenden Erfindung, soweit nicht abweichend definiert, durch Alkyl-Gruppen substituierte Aryl-Gruppen, die eine Alkylenkette aufweisen und im Arylgerüst ein oder mehrere Heteroatome, die ausgewählt sind aus O, N, P und S, aufweisen können.

Als Oxidationsmittel zur Herstellung der Sulfoxide sind Wasserstoffperoxid, Alkylhydroperoxide und Arylalkylhydroperoxide geeignet. Bevorzugtes Oxidationsmittel ist Wasserstoffperoxid. Das molare Verhältnis von Oxidationsmittel zum Sulfid liegt im Bereich von 0,9:1 bis 4:1, bevorzugt zwischen 1,2:1 und 2,5:1.

Der chirale Metall-Ligand-Komplex wird aus einem chiralen Ligand und einer Übergangsmetallverbindung hergestellt.

Übergangsmetall-Derivate sind bevorzugt Vanadium-, Molybdän-, Zirkonium-, Eisen-, Mangan- und Titan-Derivate, ganz bevorzugt Vanadium-Derivate. Diese Derivate können beispielsweise in Form der Übergangsmetall-(IV)-halogenide, Übergangsmetall-(IV)-alkoxide oder Übergangsmetall-(IV)-acetylacetonate eingesetzt werden.

Der chirale Ligand ist eine chirale Verbindung, die in der Lage ist beispielsweise mit den Vanadiumderivaten zu reagieren. Solche Verbindungen werden vorzugsweise ausgewählt aus chiralen Alkoholen. Bevorzugte chirale Liganden schliessen ebenfalls Schiff'sche Basen der Formel (III) und (IV) ein: 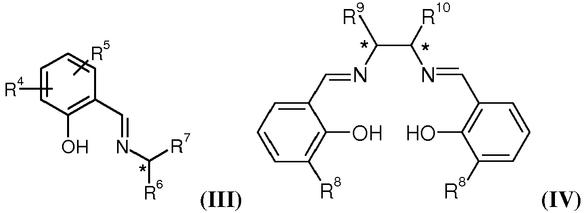 wobei in Formel (III)
R⁴ und R⁵ unabhängig voneinander für Wasserstoff, (C₁-C₆)Alkyl, (C₁-C₆)Alkylphenyl, Phenyl, Halogen, Cyano, Nitro, Cyano(C₁-C₆)alkyl, Hydroxy(C₁-C₆)alkyl, (C₁-C₆)Alkoxycarbonyl(C₁-C₆)alkyl, (C₁-C₆)Alkoxy(C₁-C₆)alkyl stehen,
R⁶ für (C₁-C₆)Alkyl, durch Halogen, Cyano, Nitro, Amino, Hydroxy oder Phenyl substituiertes (C₁-C₆)Alkyl), Carboxyl, Carbonyl(C₁-C₆)alkyl, (C₁-C₆)Alkoxycarbonyl(C₁-C₆)alkyl, (C₁-C₆)Alkoxy(C₁-C₆)alkyl, (C₁-C₆)Alkoxy, Di(C₁-C₆)alkoxy(C₁-C₆)alkyl steht,
R⁷ für Wasserstoff, (C₁-C₆)Alkyl, (C₁-C₆)Alkylphenyl, Aryl, Aryl(C₁-C₆)alkyl steht, bevorzugt für *tert*.-Butyl, Benzyl, Phenyl steht
und chirale Kohlenstoffatome mit * gekennzeichnet sind,
wobei in Formel (IV)
R⁸ für Wasserstoff, (C₁-C₆)Alkyl, (C₁-C₆)Alkylphenyl, Phenyl, Halogen, Cyano, Nitro, Cyano(C₁-C₆)alkyl, Hydroxy(C₁-C₆)alkyl, (C₁-C₆)Alkoxycarbonyl(C₁-C₆)alkyl, (C₁-C₆)Alkoxy(C₁-C₆)alkyl stehen,
R⁹ und R¹⁰ für Wasserstoff, (C₁-C₆)Alkyl, Phenyl stehen, wobei R⁹ und R¹⁰ eine Brücke bilden können und
chirale Kohlenstoffatome mit * gekennzeichnet sind.

Diese Schiff'sche Basen können einen chiralen Metall-Ligand-Komplex bilden, der als chiraler (Salen)-Metallkomplex bekannt ist. Die Stöchiometrie der chiralen Komplexe kann variieren und ist für die Erfindung nicht kritisch.

Der Einsatz von chiralem Metall-Ligand-Komplex im Vergleich zum Sulfid liegt im Bereich bei 0,001 bis 10 mol%, bevorzugt von 0,1 bis 5 mol%, ganz besonders bevorzugt 1 bis 4 mol%. Ein höherer Einsatz an chiralem Metall-Ligand-Komplex ist möglich, aber wirtschaftlich nicht sinnvoll.

Der chirale Übergangsmetallkomplex wird durch Reaktion eines Übergangsmetall-Derivates und einem chiralen Komplexliganden separat oder in Gegenwart des Sulfides erzeugt.

Die Umsetzung des Sulfides der Formel (II) zur Verbindung mit der Formel (I) kann in Gegenwart eines Lösungsmittels durchgeführt werden. Als geeignete Lösungsmittel sind insbesondere zu nennen: THF, Dioxan, Diethylether, Diglyme, Methyl-tert-butylether (MTBE), tert-Amylmethylether (TAME), Dimethylether (DME), 2-Methyl-THF, Acetonitril, Butyronitril, Toluol, Xylole, Mesitylen, Ethylacetat, Isopropylacetat, Alkohole wie Methanol, Ethanol, Propanol, Butanol, Ethylenglycol, Ethylencarbonat, Propylencarbonat, N,N-Dimethylacetamid, N,N-Dimethylformamid , N-Methylpyrrolidon, Halogenkohlenwasserstoffe und aromatische Kohlenwasserstoffe, insbesondere Chlorkohlen-wasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Tri-chlorbenzol; 4-Methoxybenzol, fluorierte Aliphate und Aromaten wie Trichlortrifluorethan, Benzotrifluorid, 4-Chlorbenzotrifluorid, und Wasser. Es können auch Lösungsmittelgemische eingesetzt werden.

Es wurde zudem beobachtet, daß das Enantiomerenverhältnis nicht nur über das Katalysatorsystem, sondern auch über das Lösungsmittel gesteuert werden kann.

Als weitere Einflußfaktoren für das Enantiomerenverhältnis sind neben dem Oxidationsmittel auch die Temperatur zu nennen.

Geeignete Methoden zur Bestimmung des Enantiomerenüberschusses sind dem Fachmann geläufig. Als Beispiele können HPLC an chiralen stationären Phasen und NMR-Untersuchungen mit chiralen Shiftreagenzien genannt werden.

Die Umsetzung wird im allgemeinen bei einer Temperatur zwischen - 80°C und 200°C, vorzugsweise zwischen 0°C und 140°C, ganz besonders bevorzugt zwischen 10°C und 60°C, sowie bei einem Druck bis zu 100 bar, vorzugsweise bei einem Druck zwischen Normaldruck und 40 bar, durchgeführt.

Die Herstellung der Thioether der allgemeinen Formel (II) ist beispielsweise in WO 1999/055668 beschrieben oder kann analog durchgeführt werden.

Die Liganden werden nach bekannten Verfahren hergestellt (Adv. Synth. Catal. 2005, 347, 1933-1936).

Die Isolierung der gewünschten Verbindungen der allgemeinen Formel (I) kann beispielsweise durch anschließende Extraktion und Kristallisation erfolgen.

Die vorliegende Erfindung wird anhand der nachfolgenden Beispiele näher erläutert, wobei die Beispiele nicht in die Erfindung einschränkender Weise zu interpretieren sind.

Nach dem erfindungsgemäßen Verfahren anfallende Produkte weisen ein Enantiomerenverhältnis von 50,5:49,5 bis 99,5:0,5, bevorzugt von 60:40 bis 95:5, besonders bevorzugt von 75:25 bis 90:10 (+):(-)-Enantiomer oder (-):(+)-Enantiomer, ganz besonders bevorzugt (+):(-)-Enantiomer auf. Damit sind in den angegebenen Bereichen diejenigen Enantiomerenverhältnisse erfindungsgemäß bevorzugt, welche einen Überschuss am (+)-Enantiomer aufweisen.

Somit kann der Enantiomerenüberschuß zwischen 0% ee und 99% ee liegen. Der Enantiomerenüberschuss ist ein indirektes Maß für die Enantiomerenreinheit einer Verbindung und gibt den Anteil eines reinen Enantiomers in einem Gemisch, dessen übriger Teil das Racemat der Verbindung ist.

Bei Bedarf kann durch eine nachfolgende Kristallisation mit oder ohne Lösungsmittel der Enantiomerenüberschuß erheblich gesteigert werden. Solche Verfahren sind dem Fachmann bekannt und schließen insbesondere die bevorzugte Kristallisation aus einem organischen Lösungsmittel oder einem Gemisch aus organischem Lösungsmittel mit Wasser ein.

### Herstellungbeispiele:

### Beispiel 1: Synthese von (+)-1-{2,4-Dimethyl-5-[(2,2,2-trifluorethyl)sulfinyl]phenyl}-3-(trifluormethyl)-1H-1,2,4-triazol

In einem Dreihalskolben wurden 10,3 g (27,54 mmol, 95%ig) 1-{2,4-Dimethyl-5-[(2,2,2-trifluorethyl)sulfanyl]phenyl}-3-(trifluormethyl)-1H-1,2,4-triazol und 145,8 mg (0,55 mmol) Vanadiumacetylacetonat in 36 ml Chloroform gelöst und 10 Minuten gerührt. Anschliessend wurden 275,8 mg (0,825 mmol) (S)-(2,4-Di-*tert*.-butyl-6-{(E)-[(1-hydroxy-3,3-dimethylbutan-2-yl)imino]methyl}phenol zugesetzt. Nach 10 Minuten wurden 5,66 g (50 mmol) 30%iger H₂O₂ über 6 Stunden zudosiert. Der Verlauf der Umsetzung wurde mittels HPLC verfolgt. Nach 4h Reaktionszeit wurden weitere 145,8 mg (0,55 mmol) Vanadiumacetylacetonat und 275,8 mg (2,4-Di-tert-butyl-6-{(E)-[(1-hydroxy-3,3-dimethylbutan-2-yl)imino]methyl}phenol in 4 ml Chloroform zudosiert. Anschließend gab man nacheinander 40 ml Chloroform, 20 ml Wasser sowie 20 ml Thiosulfatlösung. Nach dem Abtrennen der wässrigen Phase wurde die organische Phase mit Wasser gewaschen, über Na₂SO₄ getrocknet und das Lösungsmittel im Vakuum verdampft. Man erhielt 10,84 g graubraune Kristalle von (+)-1-{2,4-Dimethyl-5-[(2,2,2-trifluorethyl)-sulfinyl]phenyl}-3-(trifluormethyl)-1H-1,2,4-triazol (98 % Ausbeute, 93,1% HPLC-Reinheit) mit 2,81% Sulfonanteil. Der Enantiomerenüberschuß wurde mittels HPLC an chiraler Phase (Daicel Chiracel OJ-RH 150) mit einem Verhältnis von 16,34 : 83,66 bestimmt.

Das Enantiomerenverhältnis wurde, beispielsweise durch Kristallisation aus CHCl₃, auf 3,39:96,61 verbessert.

**Tabelle 1: Oxidation von 1-{2,4-Dimethyl-5-[(2,2,2-trifluorethyl)sulfanyl]phenyl}-3-(trifluor-methyl)-1H-1,2,4-triazol unter unterschiedlichen Bedingungen:**

| **Oxidationsmittel** | **Lösungsmittel** | **Enantiomerenverhältnis** [(-):(+)] |
|---|---|---|
| H₂O₂ | CHCl₃ | 16,34:83,66 |
| H₂O₂ | CHCl₃ | 3,39:96,61 |
| | | nach Kristallisation aus CHCl₃ |
| H₂O₂ | CHCl₃:Toluol | 18,54:81,46 |
| tert.-Butylhydroperoxid | CHCl₃ | 50,0:50,0 |
| Cumolhydroperoxid | CHCl₃ | 50:50 |
| H₂O₂ | Acetonitril | 50:50 |
| H₂O₂ | DME | 31,49:68,51 |
| H₂O₂ | n-Butanol | 36,61:63,39 |
| H₂O₂ | Methanol | 50:50 |
| H₂O₂ | Eisessig | 42,58:57,42 |
| H₂O₂ | EE | 35,18:64,82 |
| H₂O₂ | THF | 50:50 |
| H₂O₂ | Me-THF | 36,0:64,0 |
| H₂O₂ | 4-Methoxybenzol | 16,33:83,67 |
| | 1,2-Dichlorbenzol | 18,71:81,29 |
| H₂O₂ | Chlorbenzol | 27,87:72,13 |
| H₂O₂ | 4-Cl-Benzotrifluorid | 24,94:75,06 |
| H₂O₂ | 1,1,2,2-Tetrachlorethan | 18,97:81,03 |
| H₂O₂ | DMAC | 46,78:53,22 |

**Tabelle 2: Oxidation von 1-{2,4-Dimethyl-5-[(2,2,2-trifluorethyl)sulfanyl]phenyl}-3-(trifluor-methyl)-1H-1,2,4-triazol mit unterschiedlichen Katalysatoren:**

| **Katalysatorsystem** | | **Oxidations -mittel** | **Lösungsmittel** | **T** | **Enantiomerenverhältnis [(-):(+)]** |
|---|---|---|---|---|---|
| VO(acac)₂ / | 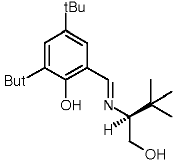 | H₂O₂ | CHCl₃ | RT | 16,34:83,66 |
| Fe(acac)₃ / | 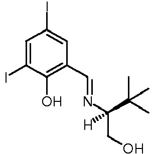 | H₂O₂ | DCM | RT | 18,04:81,96 |
| 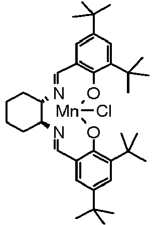 | Chiral | H₂O₂ | Acetonitril | 40 °C | 19,91:80,09 |

### Beispiel 2: 3-(Difluormethyl)-1-{2,4-dimethyl-5-[(2,2,2-trifluorethyl)sulfinyl]phenyl}-1H-1,2,4-triazol

Analog Beispiel 1 wurde aus 3-(Difluormethyl)-1-{2,4-dimethyl-5-[(2,2,2-trifluorethyl)-sulfanyl]-phenyl}-1H-1,2,4-triazol 3-(Difluormethyl)-1-{2,4-dimethyl-5-[(2,2,2-trifluorethyl)-sulfinyl]-phenyl}-1H-1,2,4-triazol erhalten. Der Enantiomerenüberschuss wurde mittels HPLC an chiraler Phase (Daicel Chiracel OJ-RH 150) mit einem Verhältnis von 7,37 : 92,63 bestimmt.

### Beispiel 3: 1-{5-[(2,2-Difluorethyl)sulfinyl]-2,4-dimethylphenyl}-3-(difluormethyl)-1H-1,2,4-triazol

Analog Beispiel 1 wurde aus 1-{5-[(2,2-Difluorethyl)sulfanyl]-2,4-dimethylphenyl}-3-(difluor-methyl)-1H-1,2,4-triazol 1-{5-[(2,2-Difluorethyl)sulimyl]-2,4-dimethylphenyl}-3-(difluormethyl)-1H-1,2,4-triazol erhalten. Der Enantiomerenüberschuss wurde mittels HPLC an chiraler Phase (Daicel Chiracel OJ-RH 150) mit einem Verhältnis von 19,97:80,03 bestimmt.

## Patentansprüche

1. Verfahren zur Herstellung von 3-Triazolyl-Sulfoxid-Derivaten der Formel (I) in enantiomerenreiner oder enantiomerenangereicherter Form, 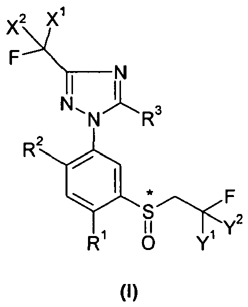 in welcher
X¹ und X² unabhängig voneinander für Fluor, Chlor, Brom, Wasserstoff, (C₁-C₁₂)Alkyl, (C₁-C₁₂)Halogenalkyl stehen,
Y¹ und Y² unabhängig voneinander für Fluor, Chlor, Brom, Wasserstoff, (C₁-C₁₂)Alkyl, (C₁-C₁₂)Halogenalkyl stehen,
R¹ und R² unabhängig voneinander für Wasserstoff, (C₁-C₁₂)Alkyl, (C₁-C₁₂)Halogenalkyl, Cyano, Halogen, Nitro stehen,
R³ für Wasserstoff, (C₁-C₁₂)Alkyl, Amino, Nitro, NH(CO)(C₁-C₁₂)Alkyl, N=CR'R steht,
wobei R, R' unabhängig voneinander für Wasserstoff, (C₁-C₁₂)Alkyl, Aryl stehen,
**dadurch gekennzeichnet, daß** man
(A) ein Sulfid der Formel (II) 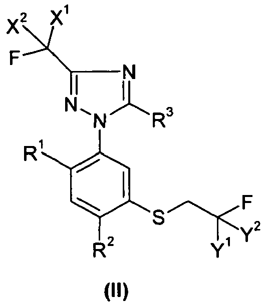 in welcher X¹, X², Y¹, Y², R¹, R² und R³ die oben genannten Bedeutungen haben,
in Gegenwart eines chiralen Katalysators und eines Oxidationsmittels, umsetzt,
wobei das Oxidationsmittel ausgewählt ist aus Wasserstoffperoxid, Alkylhydroperoxid und Arylalkylhydroperoxid und
wobei der chirale Katalysator ein chiraler Metall-Ligand-Komplex ist und das Metall ein Übergangsmetall ist.

2. Verfahren gemäß Anspruch 1, **dadurch gekennzeichnet, dass** das Enantiomerenverhältnis 50,5:49,5 bis 99,5:0,5 (+):(-) oder (-):(+)-Enantiomer beträgt.

3. Verfahren gemäß einem der Ansprüche 1 bis 2, **dadurch gekennzeichnet, dass** das Enantiomerenverhältnis 50,5:49,5 bis 99,5:0,5 (+):(-) Enantiomer beträgt.

4. Verfahren gemäß einem der Ansprüche 1 bis 3, **dadurch gekennzeichnet, dass**
X¹ und X², Y¹ und Y² unabhängig voneinander für Fluor, Chlor, Wasserstoff, (C₁-C₁₂)Halogenalkyl stehen,
R¹ und R² unabhängig voneinander für Fluor, Wasserstoff, (C₁-C₆)Alkyl stehen,
R³ für Wasserstoff, Amino steht.

5. Verfahren gemäß einem der Ansprüche 1 bis 4, **dadurch gekennzeichnet, dass**
X¹ und X², Y¹ und Y² unabhängig voneinander für Fluor, Wasserstoff, (C₁-C₆)Halogenalkyl stehen,
R¹ und R² unabhängig voneinander für Fluor, Methyl stehen,
R³ für Wasserstoff steht.

6. Verfahren gemäß einem der Ansprüche 1 bis 5, **dadurch gekennzeichnet, dass** als chiraler Katalysator ein chiraler Metall-Ligand Komplex eingesetzt wird, wobei das Metall ein Übergangsmetall ist und der Ligand eine Verbindung der Formel (III) oder (IV) darstellt, 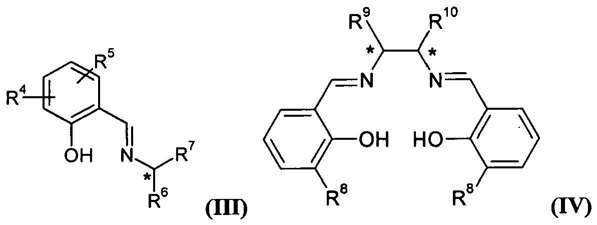 wobei in Formel (III)
R⁴ und R⁵ unabhängig voneinander für Wasserstoff, (C₁-C₆)Alkyl, (C₁-C₆)Alkylphenyl, Phenyl, Halogen, Cyano, Nitro, Cyano(C₁-C₆)alkyl, Hydroxy(C₁-C₆)alkyl, (C₁-C₆)Alkoxycarbonyl(C₁-C₆)alkyl, (C₁-C₆)Alkoxy(C₁-C₆)alkyl stehen,
R⁶ für (C₁-C₆)Alkyl, durch Halogen, Cyano, Nitro, Amino, Hydroxy oder Phenyl substituiertes (C₁-C₆)Alkyl, Carboxyl, Carbonyl(C₁-C₆)alkyl, (C₁-C₆)Alkoxycarbonyl(C₁-C₆)alkyl, (C₁-C₆)Alkoxy(C₁-C₆)alkyl, (C₁-C₆)Alkoxy, Di(C₁-C₆)alkoxy(C₁-C₆)alkyl steht,
R⁷ für Wasserstoff, (C₁-C₆)Alkyl, (C₁-C₆)Alkylphenyl, Aryl, Aryl(C₁-C₆)alkyl steht, bevorzugt für *tert*.-Butyl, Benzyl, Phenyl steht
und chirale Kohlenstoffatome mit * gekennzeichnet sind,
wobei in Formel (IV)
R⁸ für Wasserstoff, (C₁-C₆)Alkyl, (C₁-C₆)Alkylphenyl, Phenyl, Halogen, Cyano, Nitro, Cyano(C₁-C₆)alkyl, Hydroxy(C₁-C₆)alkyl, (C₁-C₆)Alkoxycarbonyl(C₁-C₆)alkyl, (C₁-C₆)Alkoxy(C₁-C₆)alkyl stehen,
R⁹ und R¹⁰ für Wasserstoff, (C₁-C₆)Alkyl, Phenyl stehen, wobei R⁹ und R¹⁰ eine Brücke bilden können und
chirale Kohlenstoffatome mit * gekennzeichnet sind.

7. Verfahren gemäß einem der Ansprüche 1 bis 6, **dadurch gekennzeichnet, dass** im Anschluß an den Schritt (A) eine Kristallisation aus organischem Lösungsmittel oder einem Gemisch aus organischem Lösungsmittel mit Wasser durchgeführt wird.

8. Enantiomerenreine oder enantiomerenangereicherte 3-Triazolyl-Sulfoxid-Derivate der Formel (I), herstellbar nach dem Verfahren gemäß einem der Ansprüche 1 bis 7, wobei das Enantiomerenverhältnis 50,5:49,5 bis 99,5:0,5 (+):(-)Enantiomer beträgt.

## Claims

1. Process for preparing 3-triazolyl sulphoxide derivatives of the formula (I) in an enantiomerically pure or enantiomerically enriched form 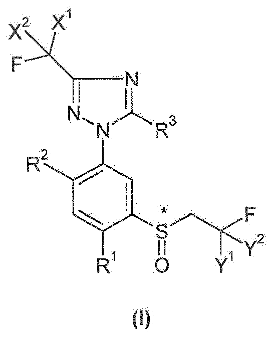 in which
X¹ and X² are each independently fluorine, chlorine, bromine, hydrogen, (C₁-C₁₂)alkyl, (C₁-C₁₂)haloalkyl,
Y¹ and Y² are each independently fluorine, chlorine, bromine, hydrogen, (C₁-C₁₂) alkyl, (C₁-C₁₂)haloalkyl,
R¹ and R² are each independently hydrogen, (C₁-C₁₂)alkyl, (C₁-C₁₂)haloalkyl, cyano, halogen, nitro,
R³ is hydrogen, (C₁-C₁₂)alkyl, amino, nitro, NH (CO) (C₁-C₁₂)alkyl, N=CR'R
where R, R' are each independently hydrogen, (C₁-C₁₂)alkyl, aryl,
**characterized in that**
(A) a sulphide of the formula (II) 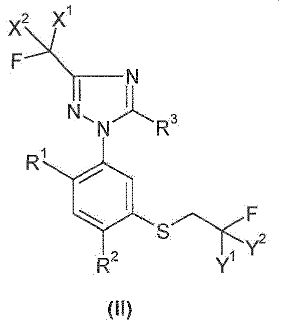 in which X¹, X², Y¹, Y², R¹, R² and R³ are each as defined above
is converted in the presence of a chiral catalyst and of an oxidizing agent,
where the oxidizing agent is selected from hydrogen peroxide, alkyl hydroperoxide and arylalkyl hydroperoxide and
where the chiral catalyst is a chiral metal-ligand complex and the metal is a transition metal.

2. Process according to Claim 1, **characterized in that** the enantiomer ratio is 50.5:49.5 to 99.5:0.5 (+) : (-) or (-) : (+) -enantiomer.

3. Process according to either of Claims 1 and 2, **characterized in that** the enantiomer ratio is 50.5:49.5 to 99.5:0.5 (+):(-) enantiomer.

4. Process according to any of Claims 1 to 3, **characterized in that**
X¹ and X², Y¹ and Y² are each independently fluorine, chlorine, hydrogen, (C₁-C₁₂)haloalkyl,
R¹ and R² are each independently fluorine, hydrogen, (C₁-C₆)alkyl,
R³ is hydrogen, amino.

5. Process according to any of Claims 1 to 4, **characterized in that**
X¹ and X², Y¹ and Y² are each independently fluorine, hydrogen, (C₁-C₆)haloalkyl,
R¹ and R² are each independently fluorine, methyl,
R³ is hydrogen.

6. Process according to any of Claims 1 to 5, **characterized in that** the chiral catalyst used is a chiral metal-ligand complex, where the metal is a transition metal and the ligand is a compound of the formula (III) or (IV) 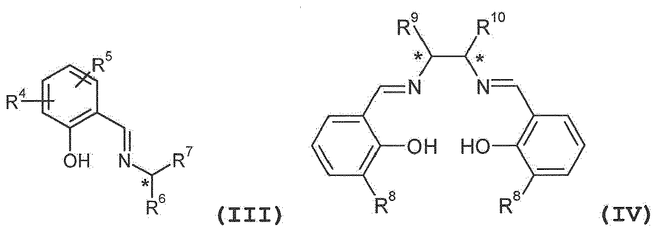 where, in formula (III),
R⁴ and R⁵ are each independently hydrogen, (C₁-C₆)alkyl, (C₁-C₆)alkylphenyl, phenyl, halogen, cyano, nitro, cyano(C₁-C₆)alkyl, hydroxy (C₁-C₆)alkyl, (C₁-C₆)alkoxycarbonyl(C₁-C₆)alkyl, (C₁-C₆)alkoxy(C₁-C₆)alkyl,
R⁶ is (C₁-C₆)alkyl, halogen-, cyano-, nitro-, amino-, hydroxyl- or phenyl-substituted (C₁-C₆)alkyl, carboxyl, carbonyl (C₁-C₆)alkyl, (C₁-C₆)alkoxycarbonyl (C₁-C₆)alkyl, (C₁-C₆)alkoxy(C₁-C₆)alkyl, (C₁-C₆)alkoxy, di (C₁-C₆)alkoxy(C₁-C₆)alkyl,
R⁷ is hydrogen, (C₁-C₆)alkyl, (C₁-C₆)alkylphenyl, aryl, aryl(C₁-C₆)alkyl, preferably *tert-*butyl, benzyl, phenyl,
and chiral carbon atoms are designated *,
where, in formula (IV),
R⁸ is hydrogen, (C₁-C₆)alkyl, (C₁-C₆)alkylphenyl, phenyl, halogen, cyano, nitro, cyano(C₁-C₆)alkyl, hydroxy (C₁-C₆)alkyl, (C₁-C₆)alkoxycarbonyl (C₁-C₆)alkyl, (C₁-C₆)alkoxy (C₁-C₆) alkyl,
R⁹ and R¹⁰ are each hydrogen, (C₁-C₆)alkyl, phenyl, where R⁹ and R¹⁰ may form a bridge, and
chiral carbon atoms are designated *.

7. Process according to any of Claims 1 to 6, **characterized in that** step (A) is followed by performing a crystallization from organic solvent or a mixture of organic solvent with water.

8. Enantiomerically pure or enantiomerically enriched 3-triazolyl sulphoxide derivatives of the formula (I), preparable by the process according to any of Claims 1 to 7, where the enantiomer ratio is 50.5:49.5 to 99.5:0.5 (+):(-)enantiomer.

## Revendications

1. Procédé de fabrication de dérivés de 3-triazolyl-sulfoxyde de formule (I) sous forme énantiomériquement pure ou énantiomériquement enrichie 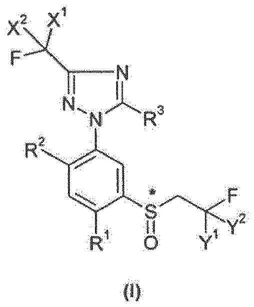 dans laquelle
X¹ et X¹ représentent indépendamment l'un de l'autre fluor, chlore, brome, hydrogène, alkyle en (C₁-C₁₂), halogénoalkyle en (C₁-C₁₂),
Y¹ et Y² représentent indépendamment l'un de l'autre fluor, chlore, brome, hydrogène, alkyle en (C₁-C₁₂), halogénoalkyle en (C₁-C₁₂),
R¹ et R² représentent indépendamment l'un de l'autre hydrogène, alkyle en (C₁-C₁₂), halogénoalkyle en (C₁-C₁₂), cyano, halogène, nitro,
R³ représente hydrogène, alkyle en (C₁-C₁₂), amino, nitro, NH(CO)alkyle en (C₁-C₁₂), N=CR'R,
R, R' représentant indépendamment l'un de l'autre hydrogène, alkyle en (C₁-C₁₂), aryle,
**caractérisé en ce que**
(A) un sulfure de formule (II) 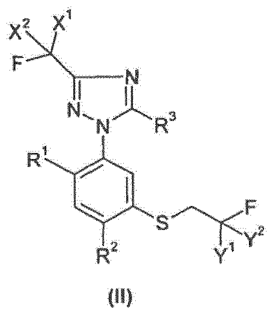 dans laquelle X¹, X², Y¹, Y², R¹, R² et R³ ont les significations indiquées précédemment,
est mis en réaction en présence d'un catalyseur chiral et d'un oxydant,
l'oxydant étant choisi parmi le peroxyde d'hydrogène, l'hydroperoxyde d'alkyle et l'hydroperoxyde d'arylalkyle, et
le catalyseur chiral étant un complexe métal-ligand chiral et le métal étant un métal de transition.

2. Procédé selon la revendication 1, **caractérisé en ce que** le rapport entre les énantiomères (+):(-) ou (-) : (+) est de 50,5:49,5 à 99,5:0,5.

3. Procédé selon l'une quelconque des revendications 1 à 2, **caractérisé en ce que** le rapport entre les énantiomères (+):(-) est de 50,5:49,5 à 99,5:0,5.

4. Procédé selon l'une quelconque des revendications 1 à 3, **caractérisé en ce que**
X¹ et X², Y¹ et Y² représentent indépendamment les uns des autres fluor, chlore, hydrogène, halogénoalkyle en (C₁-C₁₂),
R¹ et R² représentent indépendamment l'un de l'autre fluor, hydrogène, alkyle en (C₁-C₆),
R³ représente hydrogène, amino.

5. Procédé selon l'une quelconque des revendications 1 à 4, **caractérisé en ce que**
X¹ et X², Y¹ et Y² représentent indépendamment les uns des autres fluor, hydrogène, halogénoalkyle en (C₁-C₆),
R¹ et R² représentent indépendamment l'un de l'autre fluor, méthyle,
R³ représente hydrogène.

6. Procédé selon l'une quelconque des revendications 1 à 5, **caractérisé en ce qu'**un complexe métal-ligand chiral est utilisé en tant que catalyseur chiral, le métal étant un métal de transition et le ligand étant un composé de formule (III) ou (IV) 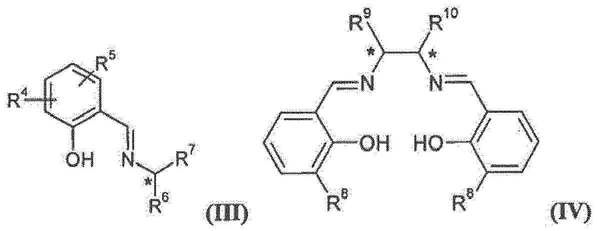 dans la formule (III)
R⁴ et R⁵ représentant indépendamment l'un de l'autre hydrogène, alkyle en (C₁-C₆), alkylphényle en (C₁-C₆), phényle, halogène, cyano, nitro, cyano-alkyle en (C₁-C₆), hydroxy-alkyle en (C₁-C₆), alcoxycarbonyle en (C₁-C₆)-alkyle en (C₁-C₆), alcoxy en (C₁-C₆)-alkyle en (C₁-C₆),
R⁶ représentant alkyle en (C₁-C₆) ; alkyle en (C₁-C₆) substitué par halogène, cyano, nitro, amino, hydroxy ou phényle ; carboxyle, carbonyl-alkyle en (C₁-C₆), alcoxycarbonyle en (C₁-C₆)-alkyle en (C₁-C₆), alcoxy en (C₁-C₆)-alkyle en (C₁-C₆), alcoxy en (C₁-C₆), di-alcoxy en (C₁-C₆)-alkyle en (C₁-C₆),
R⁷ représentant hydrogène, alkyle en (C₁-C₆), alkylphényle en (C₁-C₆), aryle, aryl-alkyle en (C₁-C₆),
de préférence *tert*.-butyle, benzyle, phényle,
et les atomes de carbone chiraux étant **caractérisés par** *,
dans la formule (IV),
R⁸ représentant hydrogène, alkyle en (C₁-C₆), alkylphényle en (C₁-C₆), phényle, halogène, cyano, nitro, cyano-alkyle en (C₁-C₆), hydroxy-alkyle en (C₁-C₆), alcoxycarbonyle en (C₁-C₆)-alkyle en (C₁-C₆), alcoxy en (C₁-C₆)-alkyle en (C₁-C₆),
R⁹ et R¹⁰ représentant hydrogène, alkyle en (C₁-C₆), phényle, R⁹ et R¹⁰ pouvant former un pont, et
les atomes de carbone chiraux étant **caractérisés par** *.

7. Procédé selon l'une quelconque des revendications 1 à 6, **caractérisé en ce qu'**une cristallisation à partir d'un solvant organique ou d'un mélange d'un solvant organique avec de l'eau est réalisée après l'étape (A).

8. Dérivés de 3-triazolyl-sulfoxyde de formule (I) énantiomériquement purs ou énantiomériquement enrichis, pouvant être fabriqués par le procédé selon l'une quelconque des revendications 1 à 7, le rapport entre les énantiomères (+):(-) étant de 50,5:49,5 à 99,5:0,5.
